# EUROPEAN PATENT APPLICATION

(11) **EP 3 628 390 A1**
(43) Date of publication of application: **01.04.2020**
(21) Application number: 19199168.6
(22) Date of filing: 24.09.2019
(51) Int. Cl.: B01D 53/04, B01D 53/14, B01D 53/22, C12M 1/00, C10L 3/10

(54) **BIOGAS TREATMENT ASSEMBLY**

(30) Priority: 26.09.2018 IT 201800008942
(71) Applicant: Hysytech S.r.l., 10135 Torino (IT)
(72) Inventor: ANTONINI, Massimiliano, 10064 PINEROLO (IT); SOLARO, Simone, 14055 COSTIGLIOLE D'ASTI (IT); SALDIVIA, Andres, 10138 TORINO (IT); FARINA, Corrado, 10064 PINEROLO (IT); MARCHISIO, Luigi, 10129 TORINO (IT); IELPO, Gaetano, 85100 POTENZA (IT)
(74) Representative: Pallini Gervasi, Diego

(57) **Abstract**

The invention refers to a biogas treatment assembly (1) comprising a biogas supplying line (10), by means of which the methane reaches a first treatment unit (10). The latter, in turn, comprises an absorption column (4), in which a stream of operating liquid (preferably water) dissolves part of the carbon dioxide contained in the biogas in order to obtain a flow of treated biogas depleted of carbon dioxide and enriched in methane. The first unit (10) further comprises a stripping column (5) for separating the carbon dioxide absorbed by the operating liquid in the first column (4), following a heating of the operating liquid itself. Said second column (5) generating a flow of OFFGAS with a high concentration of carbon dioxide.

## Description

### FIELD OF THE INVENTION

The present invention relates to the field of apparatuses and systems for the treatment of biogas. In particular, the present invention relates to a biogas treatment assembly configured to extract a flow of biomethane and a flow of OFFGAS with a high concentration of carbon dioxide from said biogas.

### BACKGROUND ART

As known, biogas is generated by means of fermentation processes developed in digesters or also by means of uptake processes implemented in landfills. It is also known that biogas is a mixture formed prevalently of methane (CH₄) and carbon dioxide (CO₂), and to a lesser extent (i.e. in a percentage lower than 10%) also of nitrogen and oxygen. Biogas typically comprises many other types of gases, such as ammonia, fluorine compounds, volatile organic solvents (VOCs), as well as other impurities, such as hydrogen sulphide, water, aromatic compounds and solid particles.

Over the years, various chemical and physical processes have been developed to treat biogas and more precisely to remove the carbon dioxide, the other gases and the various impurities in order to increase the percentage of methane. As a whole, the removed gas components and/or those which may be introduced to promote the separation of carbon dioxide form a gas flow indicated by the expression "process OFFGAS". In this regard, it is known that carbon dioxide can be potentially recovered/exploited in the context of methanation reactions to obtain methane or selective reduction reactions for the production of simple hydrocarbons available in the basic chemistry (building blocks). The possibility of recovering carbon dioxide is an advantageous aspect as it further justifies the investment costs for the treatment plant. Therefore, it is preferable that the aforesaid OFFGAS has a high concentration of carbon dioxide.

A first known technology is that of Pressure Swing Adsorption (PSA), in which the process of separating carbon dioxide takes place in a column in which adsorbent material, such as activated carbon or zeolites, is placed. This technology provides the positioning of several columns working in parallel. The carbon dioxide is retained by the absorbent material in the columns at high pressure (1-6 bar) and released again in the columns at low pressure (between 0.0002 and 1 bar). The columns alternate the pressure state when the saturation of the absorbent material is reached. Therefore, this process provides the implementation of (high-pressure) adsorption cycles alternating with (low-pressure) release cycles of carbon dioxide.

Although this technology is relatively easy to implement, the biogas must be pretreated before the column treatment process in order to eliminate hydrogen sulphide and the water to avoid corrosion problems. Typically, the biogas is pretreated by means of activated carbon devices which retain the aforementioned mixtures of gases which are harmful for the adsorbent material placed in the aforesaid columns. The use of activated carbon, or other functionally equivalent means, is critical in terms of cost and management. In this regard, a device must always be provided for activated carbons to constantly cool the biogas flow in order to eliminate most of the humidity with a consequent increase in energy expenditure. Activated carbons must then be replaced with a fairly high frequency. In addition to the associated installation costs, this also implies high disposal costs.

A second well-known technology provides the separation of CO₂ by means of Pressurized Water Scrubbing (PWS). This technology provides the use of two treatment columns, one for the dissolution of carbon dioxide in water and the second for the stripping of the carbon dioxide itself and its subsequent evacuation. The dissolution and stripping of carbon dioxide are two processes which depend on the physical conditions (pressure and temperature) at which they occur. Typically, carbon dioxide stripping occurs by using pressurized air introduced into the stripping column together with the water in which the carbon dioxide is dissolved. While, on one hand, this technology has the advantage of using a low-cost solvent (water), practice has shown that the costs required by this technology are justified only for very high specific volumes. In general, this technology requires high construction and operating costs and, in general, high plant operating costs. The PWS process further generates a low-quality OFFGAS with limited potential for carbon recovery. Indeed, a high volume of air is introduced into the stripping column to strip the carbon dioxide. The air remains within the OFFGAS flow in a much higher percentage than carbon dioxide. The latter is therefore difficult to recover without large investments to further treat the OFFGAS, investments which are made even more expensive in view of the large volumes of gas at stake.

Another technology widely used to separate carbon dioxide from the biogas is the one based on the use of separation membranes, typically polymer-based. This technology exploits the principle of the different permeability of different gases when crossing the same polymeric membrane. This property obviously depends on the chemical-physical structure of the membrane, but also on the pressure difference upstream and downstream of the membrane itself.

Examples of separators based on this technology are shown and described in US patent 8,999,038 B2. Such apparatuses may comprise a single separation stage or several separation stages arranged in series. In case of a single stage, for example, the membrane is typically configured to be permeable to carbon dioxide and not to methane. In a possible variant, following its separation, the permeated gas fraction (rich in carbon dioxide) is sent back into the separation stage or sent to a further separation stage. In case of multiple separation stages, following the passage into one separation stage, the gas fraction enriched with methane (i.e. not permeated through the membranes) can be sent to the next stage to increase the degree of purity of the methane.

Although the separation membrane technology allows a high degree of purification to be obtained, on the other hand it appears to be less versatile and accompanied by various drawbacks. The main reason for this is that the separation membranes are substantially ineffective in treating biogas in real state, i.e. in the composition with which it is obtained from the aforesaid digestion and/or collection processes. Indeed, even for membranes, biogas pretreatment must be implemented in order to remove some gases from the mixture, such as, for example, ammonia, fluorine compounds and volatile organic solvents, which can severely damage the membranes and/or partially obstruct them, thus determining the inevitable replacement. Typically, a gas pretreatment is provided also for separation membranes by means of activated carbon, with the same drawbacks mentioned when commenting the PSA process.

Another process used to treat biogas is cryogenic separation. In this process, the biogas is cooled in order to liquefy the carbon dioxide. For such a cooling, a refrigeration cycle or the latent heat of vaporization of a liquefied gas, such as liquid nitrogen, is used. This process, while requiring a relatively simple plant, does not allow the separation of carbon dioxide from other gases (such as H₂S) which liquefy and freeze at temperatures below those of carbon dioxide, whereby creating ice agglomerates which are difficult to manage. The need to separate the gas from the plant, the high operating cost and the strong strategic dependence on the cryogenic gas supplier are some of the drawbacks of this technology.

On the basis of these considerations, the need arises for new technical solutions which make it possible to overcome the limits and drawbacks of the technologies described above.

Therefore, the main task of the present invention is to provide a biogas treatment assembly which makes it possible to extract a flow of methane and to obtain at the same time a flow of OFFGAS with a high concentration of carbon dioxide. As part of this task, it is an object of the present invention to provide a biogas treatment assembly which is effective even for relatively small specific volumes. It is another object to provide a biogas treatment assembly in which the separation of carbon dioxide from methane takes place through purification processes which do not require a pretreatment of the biogas aimed at eliminating harmful components for the components which implement said purification. It is yet another object of the present invention to provide a biogas treatment unit which requires a relatively low external energy input. It is yet another object of the present invention to provide a biogas treatment assembly which is effective and easy to make at competitive costs.

### SUMMARY OF THE INVENTION

The present invention relates to a biogas treatment assembly comprising a biogas supplying line and a first unit for the treatment of the biogas itself. According to the invention, the first unit comprises:
- an absorption column into which said biogas and a flow of operating liquid are conveyed in which part of the carbon dioxide contained in the biogas is dissolved, wherein said absorption column comprises at least a first outlet for exiting the treated biogas depleted of the carbon dioxide;
- a second stripping column for separating the carbon dioxide dissolved in the operating liquid inside the absorption column, wherein said stripping column comprises a first outlet for exiting a gas phase rich in carbon dioxide;
- heating means for increasing the temperature of said operating liquid conveyed into the second column to a temperature value which is higher than that of the operating liquid inside the first column so as to reduce the solubility of the carbon dioxide and promote the generation of said gas phase.

The treatment assembly according to the invention makes it possible to obtain, a flow of biomethane by means of the absorption column and simultaneously, through the stripping column, a flow of OFFGAS with a high concentration of carbon dioxide, which can be potentially recovered by means of relatively simple and uncomplicated processes (e.g. methanation or selective reduction). Advantageously, the treatment assembly can work on relatively small volumes of biogas with construction and plant costs (i.e. operating costs) equally low with respect to the solutions of the prior art (in particular with respect to the PSA and PWA processes) described above.

According to a preferred embodiment, the treatment assembly further comprises a second treatment unit to purify the biogas exiting the absorption column of the first treatment unit. Such a second unit comprises at least one separating membrane.

The use of this second treatment unit provides an additional stage of carbon dioxide separation, i.e. a stage of biomethane purification. At the same time, by means of the second treatment unit, more carbon dioxide, which flows into the OFFGAS flow, is obtained. Advantageously, the gas entering the second treatment unit has already been scrubbed (i.e. treated) in the absorption column of the first treatment unit. Therefore, many of the substances typically harmful to the integrity of membranes have been removed to the benefit of a longer life of the membranes. In contrast to the prior art, in the treatment assembly according to the invention, the use of activated carbons or other devices is therefore no longer indispensable to pretreat the gas destined for the separation membranes.

According to a first aspect, the operating liquid used in the first treatment unit is water. Alternatively, a water mixture with at least one organic solvent may be used. This solution makes it possible to limit the overall volume of the operating liquid used and ultimately to contain the energy costs related to the circulation thereof.

According to a possible embodiment, the first column is connected to the second column by means of a first hydraulic line to transfer the operating liquid, enriched with carbon dioxide, from the first column to the second column; wherein pressure reducing means are arranged along said first hydraulic line to reduce the pressure of the operating liquid exiting from said absorption column to an operating value provided for the second column.

According to an embodiment, the first column is connected to the second column by means of a second hydraulic line to transfer the operating liquid exiting the second column into the first column, while the first unit comprises cooling means for cooling the operating liquid along the second hydraulic line before its entry into the first column. As a whole, the two hydraulic lines connecting the two columns of the first treatment unit make it possible to achieve an advantageous recirculation of the operating liquid and thus to contain the volume of liquid used.

In a possible embodiment, the heating means comprise an exchanger-recovery unit to transfer part of the thermal energy from the operating liquid exiting from the second column to the operating liquid at lower temperature exiting from the first column. In this manner, the thermal requirement necessary for heating the operating liquid is advantageously reduced, i.e. the external thermal energy to be supplied to take the operating liquid to the operating conditions established for the second column.

Preferably, but not exclusively, such heating means comprise an exchanger-boiler to increase the temperature of the operating liquid used in said second column by means of a heat exchange with hot water circulating in a hydraulic line outside the second column. Preferably, upstream of such heat exchange, the water circulating in the external hydraulic line has a temperature between 75° and 90°, preferably between 80° and 90°. Preferably, but not exclusively, the water circulating in said external hydraulic line is water from the recovery of thermal waste deriving from a cogeneration plant or from the cooling of a compressor or an endothermic engine.

According to a possible embodiment, the first unit comprises a branch line of the operating liquid contained within the second column; such a branch line has an inlet and an outlet placed higher than said inlet with respect to a portion of the bottom of said second column. The exchanger-reboiler is operationally placed along such a bypass line to heat the operating liquid circulating in the bypass line itself at the expense of the temperature of the water circulating in said outer hydraulic line. Advantageously, the operating liquid moves naturally in the bypass line due to the temperature difference between the inlet (at higher temperature) and outlet (at lower temperature). The natural movement of the liquid is generated by the gas phase which is generated near the inlet of the branch line where the temperature is higher. Such a gas phase reduces the density of the liquid, which therefore rises more easily towards the outlet.

According to a further embodiment, the assembly comprises a discharge line which develops from the second column for the collection of the gas phase which is generated in the second column, in which the first unit comprises an exchanger-condenser configured to cool the gas phase coming out of the second column. Preferably, a condensate storage tank is provided along this discharge line downstream of the exchanger-condenser, when the tank is hydraulically connected to the second column to take the condensed liquid back thereto. In this manner, the operating liquid which is returned to the circuit of the first treatment unit is recovered.

### LIST OF DRAWINGS

Further features and advantages of the present invention will be clearly apparent from the description of particular embodiments of the present invention illustrated by way of non-limiting example to in the attached drawings in which:
- figure 1 is a diagrammatic view of a first embodiment of a biogas treatment assembly according to the present invention;
- figure 2 is a diagrammatic view of a second embodiment of a biogas treatment assembly according to the present invention.

In the mentioned figures, the same reference numerals and letters are used to identify the same elements or components.

### DETAILED DESCRIPTION

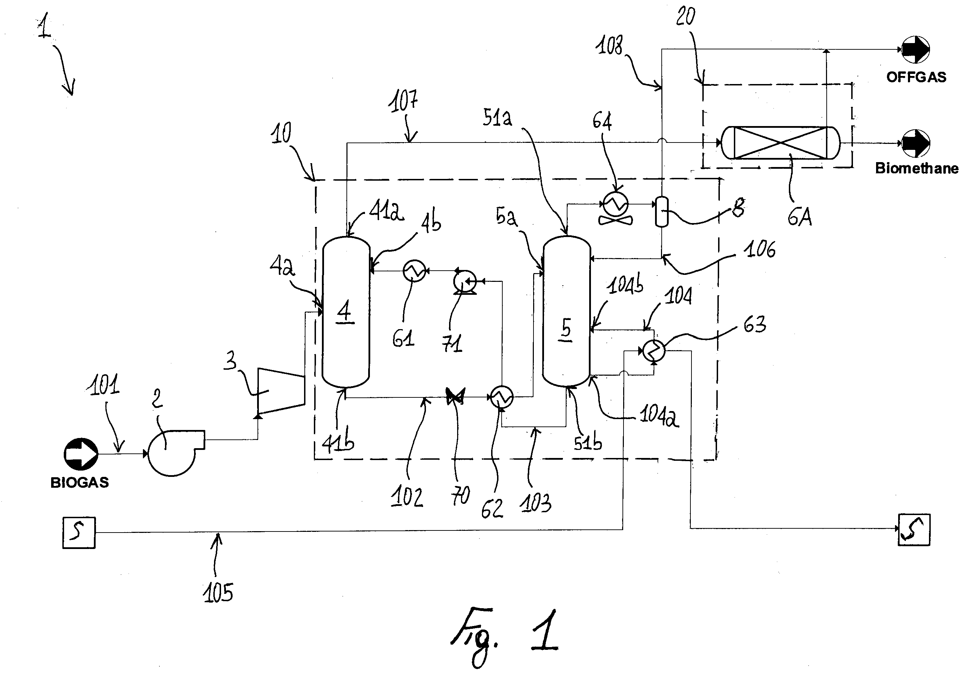

Figures 1 is a diagrammatic view of a first embodiment of a biogas treatment assembly according to the present invention indicated by reference numeral 1 as a whole. In particular, the treatment assembly 1 comprises a first treatment unit 10 configured to separate the carbon dioxide (hereinafter also indicated with the chemical formula CO₂) from the biogas, whereby increasing the percentage of methane in the biogas itself, and to recover the carbon dioxide itself in a flow of OFFGAS.

The biogas to be treated is conveyed to the first unit 10 through a supplying line 101 along which biogas suction and compression means 2, 3 are provided. Preferably, a blower 2 and a compressor 3, operationally downstream of blower 2 with respect to the direction of the biogas flow, are provided. The blower 2 aspirates biogas from a source by increasing the pressure at the inlet of compressor 3. The latter further increases the pressure value up to a first preset P1 value to introduce it into the first treatment unit 10.

According to the invention, the first unit 10 comprises a first absorption column 4 (hereinafter also referred to as absorption column 4), in which biogas is introduced and in which carbon dioxide is dissolved in a solvent liquid. Hereinafter, the solvent liquid is indicated with the expression "operating liquid" because it is the means through which carbon dioxide is removed. Most of the carbon dioxide contained in the biogas is either dissolved in the operating liquid or is absorbed by the liquid itself in the first column 4.

According to a preferred embodiment, water is chosen as operating liquid. Alternatively, a mixture of water and an organic solvent can be used as operating liquid.

The process of dissolving carbon dioxide takes place with the operating liquid at a first temperature T1 (e.g. between 25 and 30 °C if the operating liquid is water). The pressure is instead the inlet pressure P1 in the first column 4. For such a purpose, the first column 4 comprises a first inlet 4a for the introduction of biogas and a second inlet 4b for the introduction of the solvent liquid. In the first column 4, the gas phase (biogas) and the liquid phase (operating liquid) come into contact, whereby separating the carbon dioxide from the biogas and dissolving it in the operating liquid.

The first column 4 is equipped with a first outlet 41a through which the treated biogas exits, i.e. having a reduced and increased concentration of carbon dioxide compared to that of the input biogas. As a result, the treated biogas is enriched in methane compared to the input biogas.

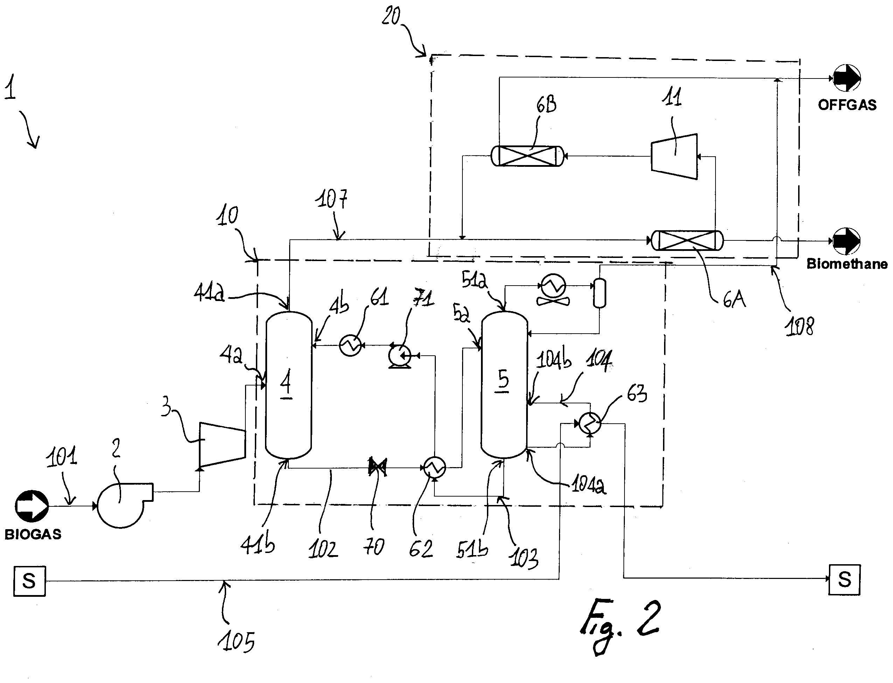

Preferably, the treated biogas is conveyed to a second treatment unit 20 which allows further purification to predefined specifications. A possible embodiment of the second treatment unit 20 is described later in the comment to Figure 2.

Filling means are present inside the first column 4 to provide a high contact surface between the gas phase and the liquid phase (operating liquid). According to a solution known in itself, bodies or groups of bodies on the surface of which the operating liquid flows may be used as filling means in order to increase the probability and duration of exposure between the two phases.

The first column 4 also comprises a second outlet 41b through which the operating liquid in which the carbon dioxide has been dissolved exits. Preferably, with respect to a vertical direction, the second outlet 41b is located near the bottom of the first column 4 where the operating liquid collects, due to the effect of gravity. On the contrary, the first outlet 41a is preferably defined near the upper part of the first column 4 where the treated biogas tends to collect.

The first treatment unit 10 further comprises a second column 5 in which the stripping of carbon dioxide from the operating liquid takes place. The second column 5 is also referred to below as *stripping column 5.* The operating liquid, once enriched with carbon dioxide during its passage in the first column, is taken into the second column 5 through a first line of circulation 102. In detail, in the second column 5, carbon dioxide is desorbed from the operating liquid to be evacuated in a gas phase destined to flow into an OFFGAS flow. The desorption (or stripping) of carbon dioxide takes place under physical conditions which are markedly different from those provided for the first column 4. More precisely, in order to reduce the solubility of carbon dioxide, the temperature of the operating liquid inside the second column 5 is taken to a value T2 higher than that (T1) in which the process is performed inside the first column 4 and at the same time the pressure in the second column 5 is taken to a value lower than that of the first column 4.

As a result of the desorption process, a gas phase is generated inside the second column 5 (rich in CO₂ separated from the operating liquid and also comprising aqueous vapor if the operating liquid is water) which exits through a first outlet 51a preferably defined near the top of the second column 5 itself, considering a vertical orientation thereof.

The first unit 10 thus comprises heating means to take the operating liquid entering the second column 5 to a temperature value of T2 (e.g. between 80 and 85 °C if the operating liquid is water) higher than that of the operating liquid in the first column 4. Pressure reduction means are also provided with the aim of reducing the water pressure to a second value P2 useful for the desorption of carbon dioxide.

According to a preferred embodiment shown in the figures, the second outlet 41b of the first column 4 is connected to a first inlet 5a of the second column 5 by means of said first hydraulic line 102. Preferably, the first inlet 5a of the second column 5 is close to the upper portion (evaluated with respect to a vertical direction) of the column itself, which is opposite to a portion of the bottom in which the operating liquid is collected by gravity.

Preferably, along the first hydraulic line 102, there are valve means 70 configured to control the water level inside the first column 4 and to reduce the water pressure to the operating pressure of the second column 5. Substantially, in this embodiment, the pressure reduction means coincide with those used for controlling the level. However, the two functions (pressure reduction and level control) could be implemented by independent means.

According to a preferred embodiment shown in the figures, the second column 5 is provided with a second outlet 51b, preferably defined near the bottom portion of the column itself (where the bottom portion is opposite to the top portion with respect to a vertical direction). The operating liquid, depleted of carbon dioxide, flows out through the second outlet 51b. The second outlet 51b is hydraulically connected to the second inlet 4b of the first column 4 by means of a second hydraulic line 103 in order to convey the liquid depleted of carbon dioxide to the first column 4 after the desorption process. Advantageously, through the two hydraulic circulation lines 102, 103, the operating liquid is continuously recirculated between the two columns 4, 5 of the first treatment unit 10.

According to an embodiment shown in the figures, the operating liquid heating means comprise an exchanger-recovery unit 62 to transfer the thermal energy of the operating liquid from the second column 5 to the operating liquid exiting the first column 4. In practice, the exchanger-recovery unit 62 allows the heating of the operating liquid entering the second column 5 (e.g. about a temperature of 60-65 °C if the operating liquid is water), by exploiting the thermal energy contained in the operating liquid exiting the same second column 5.

According to a preferred embodiment, the heating means comprise an exchanger-boiler 63 to increase the temperature of the operating liquid in the second column by means of a heat exchange with hot water circulating in a hydraulic line 105 outside the second column 5. Preferably, the operating liquid which collects at the bottom portion of the second column 5 is circulated along a branch line 104, preferably outside the second column 5, through which it returns to the column itself. More precisely, the operating liquid circulates between an inlet 104a and an outlet 104b of the branch line 104, in which said outlet 104b is located higher than said inlet 104a, with respect to a vertical reference direction. In particular, the inlet 104a is defined as a position close to the bottom of the second column 5, but above the second outlet 51b.

The exchanger-reboiler 63 is operationally arranged along the branch line 104 and is configured to heat the operating liquid circulating along the line itself at the expense of the temperature of the low-pressure hot water circulating along the outer line 105. Preferably, such hot water has a temperature between 50° and 95°, more preferably between 80° and 85°. Even more preferably, the water circulating in the outer line 105 may derive from the recovery of thermal waste from a cogeneration plant or from the cooling of compressors and/or endothermic engines.

In a less preferred embodiment, the hot water may not be waste water and therefore its temperature may be obtained through a heating device.

Inside the second column 5, the operating liquid has different temperatures according to its height. In particular, the temperature of the operating liquid increases downwards, i.e. in the direction of the bottom portion of the second column, column 5, and decreases upwards. If the operating liquid is water, at the inlet 5a, the temperature may be around 65 °C, while at the bottom (outlet 51b) the liquid may have a temperature between 80-85 °C.

As a result of this temperature profile and by effect of the mutual position of inlet 104a and outlet 104b of the branch line 104, the circulation of the operating liquid along the same occurs naturally because the liquid is pushed upwards where the temperature is lower. This circulation is also promoted by the gas phase which is generated at the inlet of the branch line 104 and which makes the liquid less dense and promotes the ascent towards the outlet 104b.

As indicated above, the operating liquid is heated by means of the exchanger-boiler 63 when passing through the branch line 104. The operating liquid thus heated along the branch line 104, once reintroduced into the second column 5, is mixed with the preheated liquid by the exchanger 62, whereby increasing the temperature and thus promoting the desorption process, i.e. the separation of carbon dioxide from the operating liquid itself.

As indicated above, through the first outlet 51a, the gas phase generated inside the second 5 can escape from it and remain conveyed along a discharge line 108. In addition to carbon dioxide, the gas phase will reasonably comprise other gases including water vapor.

According to a possible embodiment (shown in the figures), an exchanger-condenser 64 configured to cool the gas phase and at the same time to condense the water vapor contained in it is provided along the discharge line 108. For this purpose, a condensate storage tank 8 is provided immediately downstream of the condenser-exchanger 64. Advantageously, the tank 8 is hydraulically connected to the second column 5, through a return line 106, so as to take the condensate back into it. After cooling and condensation, the gas phase forms a process OFFGAS with a high concentration of carbon dioxide according to with the purposes of the invention.

According to a further aspect, the first treatment unit 10 comprises an exchanger-cooler 61 placed along the second hydraulic line 103 and downstream of the exchanger-recovery 62 mentioned above. The exchanger-cooler 61 has the function of further cooling the operating liquid in the second line 103 up to the operating value expected for the first column 4. Such cooling can occur by means of a heat exchange with air.

In order to increase the water pressure value intended for the first column 4, a circulation pump 71 is provided instead, e.g. between the exchanger-recovery unit 62 and the exchanger-cooler 61.

According to the invention, the assembly 1 comprises a second treatment unit 20 provided with one or more separation membranes 6A through which the "scrubbed" (i.e. treated) gas, coming out of the first column 4, is further purified to eliminate substances and/or gases other than methane. In this regard, the "scrubbed" gas exiting the first column 4 is taken to the second unit 20 by means of an additional hydraulic line 107.

In particular, the number of membranes and their physical structure may vary according to the degree of purity imposed on biomethane by network specifications. The membranes 6A can be operatively arranged in series and have a polymeric structure so as to be permeable to gases other than biomethane. The latter will be advantageously enriched in percentage. Through the second treatment unit 20 a flow of biomethane will be thus obtained, while the gases separated by the structure of the membranes 6 can be mixed, along the discharge line 108, with the gas phase (formed mainly by carbon dioxide) released in the second column 5 of the first treatment unit 10. As a whole, the gases separated with the membrane 6A and said gas phase will constitute the flow of OFFGAS.

According to a possible embodiment shown in Figure 2, the second treatment unit 20 may comprise at least one first membrane 6A and at least one second membrane 6B. The first membrane 6A has the function of treating the scrubbed gas coming out of the first column 4 in order to obtain a flow of biomethane at predetermined specifications. The second membrane 6B has the function of treating the gases (destined to become OFFGAS) separated through the first membrane 6A. In particular, the second membrane 6B has the function of recovering as much methane as possible or of reducing the quantity of residual methane present in OFFGAS. In this manner, the OFFGAS flows may be released into the atmosphere with minimal environmental impact, especially with regard to the greenhouse effect.

With reference to the second treatment unit 20, the configuration shown in Figure 1 is preferable if the OFFGAS flow is intended for a combustion plant or a methane plant. In this case, indeed, the residual percentage of methane (inevitably present in OFFGAS) is not a real problem because the methane is still recovered or exploited. On the contrary, if no application is provided to treat/exploit the OFFGAS, then the configuration in Figure 2 is certainly preferable, as it is aimed at minimizing the percentage of methane released into the atmosphere.

Again, with reference to Figure 2, between the first membrane 6A and the second membrane 6B, a compressor 11 is preferably provided, with the aim of increasing the pressure of the separated gases through the first membrane 6A up to the operating value required by the second membrane 6B. The second membrane 6B is therefore impermeable to methane, which is preferably mixed with the scrubbed gas coming out of the absorption column 4. In this manner, this fraction of methane is returned to the inlet of the first 6A membrane again. The methane separated through the second membrane 6B has a lower purity than that of the biomethane exiting the first membrane 6A. The second membrane 6B exploits the pressure generated by the compressor 11 to recover as much methane as possible, regardless of its purity.

In a possible embodiment, a filtering module (not shown) with activated carbon may be placed along the hydraulic line 107 and upstream of the second treatment unit 20 having the function of protecting the integrity of the membranes 6A-6B mentioned above. The purpose of such a filter module is therefore just to protect the membranes from rapid deterioration due to harmful residual compounds. The filter module therefore does not perform a process function (i.e. it does not perform a gas pretreatment of the type currently used in membrane systems).

## Claims

**1.** A biogas treatment assembly (1) comprising a line (101) for supplying said biogas and a first unit (10) for treating said biogas, **characterized in that** said first unit (10) comprises:
• an absorption column (4) to which said biogas is conveyed at the inlet together with a flow of operating liquid in which part of the carbon dioxide contained in said biogas is dissolved, wherein said absorption column (4) comprises at least a first outlet (4b) for exiting the treated biogas depleted of said carbon dioxide;
• a stripping column (5) for separating the carbon dioxide dissolved in said operating liquid inside said absorption column (4), wherein said stripping column (5) comprises at least a first outlet (5b) for exiting a gas phase rich in carbon dioxide;
• heating means for increasing the temperature of said operating liquid conveyed into said stripping column (5) to a temperature value which is higher than that of the operating liquid inside said absorption column (4) so as to reduce the solubility of the carbon dioxide and promote the generation of said gas phase.

**2.** An assembly (1) according to claim 1, **characterized in that** it comprises a second treatment unit (20) for purifying the treated biogas exiting from said absorption column (4) of said first unit (10) and wherein said second unit (20) comprises at least one separation membrane (6A).

**3.** An assembly (1) according to claim 1, **characterized in that** said operating liquid is water.
An assembly (1) according to any one of claims 1 to 3, wherein said absorption column (4) is connected to said stripping column (5) by means of a first hydraulic line (102) to transfer said operating liquid from said absorption column (4) to said stripping column (5), and wherein said first unit (10) comprises pressure reducing means (70) arranged along said first hydraulic line (102) to reduce the pressure of the operating liquid exiting from said absorption column (4) to an operating value provided for said stripping column (5).

**5.** An assembly (1) according to any one of claims 1 to 4, wherein said absorption column (4) is connected to said stripping column (5) by means of a second hydraulic line (103) to transfer the operating liquid exiting from the stripping column (5) into the absorption column (4), and wherein said first unit (10) comprises cooling means for cooling the water running along said second hydraulic line (103) before it enters into said absorption column (4).

**6.** An assembly (1) according to claim 5, wherein said heating means comprise an exchanger-recovery unit (62) to transfer part of the thermal energy from the operating liquid exiting from said stripping column (5) to the operating liquid exiting from said absorption column (4).

**7.** An assembly (1) according to any one of claims 1 to 6, wherein said heating means comprise an exchanger-reboiler (63) to increase the temperature of said operating liquid used in said stripping column (5) by means of a heat exchange with hot water circulating in a hydraulic line (105) outside said second column (5).

**8.** An assembly (1) according to claim 7, wherein in said external hydraulic line (105), the water for the recovery of thermal waste of a co-generation system or the cooling water of a compressor or the cooling water of an endothermic engine circulates.

**9.** An assembly (1) according to claim 7 or 8, wherein said first unit (10) comprises a branch line (104) of the water collected inside said stripping column (5), said exchanger-reboiler (63) being operatively arranged along the branch line (104) to heat the operating liquid circulating in said branch line (104) to the detriment of the temperature of the water circulating in said external hydraulic line (105).

**10.** An assembly (1) according to any one of claims 1 to 9, wherein said assembly (1) comprises a discharge line (108) to collect the gas phase which is generated in said stripping column (5), said discharge line (108) developing from said stripping column (5), and said first unit (10) comprising an exchanger-condenser (64) installed along the discharge line (108) and configured to cool said gas phase exiting from said stripping column (5).

**11.** An assembly (1) according to any one of claims 1 to 10, wherein said second treatment unit (20) comprises a first membrane (6A) of several membranes to treat the biogas exiting from said absorption column (4) and at least a second membrane (6B) to treat the gases separated through said first membrane (6A), wherein said second unit (20) preferably comprises a compressor (11) to increase the pressure of the gases addressed to said second membrane (6B).
